# EUROPEAN PATENT APPLICATION

(11) **EP 2 984 944 A1**
(43) Date of publication of application: **17.02.2016**
(21) Application number: 14761119.8
(22) Date of filing: 07.03.2014
(51) Int. Cl.: A23L 33/17, A61K 9/08, A61K 31/198, A61K 47/12, A61P 3/04, A61P 39/06

(54) **AQUEOUS LIQUID COMPOSITION INCLUDING HIGH CONCENTRATION OF L-HISTIDINE**

(30) Priority: 07.03.2013 JP 2013045654
(71) Applicant: Ajinomoto Co., Inc., Tokyo, 104-8315 (JP)
(72) Inventor: SHIMAZU, Tsukasa, Kawasaki-shi Kanagawa 210-8681 (JP); YAMAGUCHI, Susumu, Kawasaki-shi Kanagawa 210-8681 (JP)
(74) Representative: Strehl Schübel-Hopf & Partner
(86) International application number: PCT/JP2014/055996
(87) International publication number: WO 2014/136944

(57) **Abstract**

The present invention aims to provide an aqueous liquid composition containing histidine at a high concentration, which is preferable for oral ingestion, a production method of the liquid composition, use of the liquid composition and the like.

Provided are an aqueous liquid composition containing histidine at a concentration more than 20 wt%, particularly an aqueous liquid composition further containing a divalent or higher valent organic acid, a production method of an aqueous liquid composition comprising histidine, comprising a step of dissolving histidine at a concentration of more than 20 wt% in an acidic aqueous solution containing a divalent or higher valent organic acid, and a food or drink containing the liquid composition.

## Description

### Technical Field

The present invention relates to an aqueous liquid composition containing L-histidine, a production method of the liquid composition, use of the liquid composition and the like.

### Background Art

In recent years, various physiological functions of amino acid have been clarified, and many foods, drinks and the like containing amino acid have been placed in the market. Drinks and liquids containing amino acid are known to show precipitation and sedimentation when they contain amino acid at a high concentration, and the production methods of foods and drinks containing amino acid at a high concentration have been studied and developed. For example, patent document 1 discloses a production method of an amino acid-containing food, which includes dissolving a high concentration of amino acid in hot water at 40 - 100°C adjusted to pH 3.0 - 6.0. The document describes that a food containing amino acid within the range of 0.1 - 20 wt% can be produced, and discloses a drink containing an amino acid mixture having an amino acid concentration of 1.5 wt% in the Examples. Patent document 2 discloses a production method of a liquid for medicaments or health beverages, which contains high concentrations of three kinds of branched chain amino acids of isoleucine, leucine and valine, comprising dissolving the branched chain amino acids in water in the presence of an organic acid and/or inorganic acid. In the Examples of the document, a liquid having a branched chain amino acid concentration of 4.0 g/61 - 94 mL is disclosed.

Histidine, which is one of the essential amino acids, is also recognized as a food additive, and contained in many foods. As physiological functions of histidine, for example, antioxidant action, obesity prevention and the like are known. As a composition containing histidine, patent document 3 discloses a composition having a histidine or histidine hydrochloride blending ratio of 15 - 95 parts by weight relative to solid content 100 and containing a pH adjuster and/or a corrigent such that a 1 wt% aqueous solution based on histidine has pH 5.0 - 7.5.

### [Document List]

### [patent documents]

patent document 1: JP-A-2001-145
patent document 2: JP-A-2003-212766
patent document 3: JP-A-2006-137706

### SUMMARY OF THE INVENTION

### Problems to be Solved by the Invention

When food, drink, supplement and the like containing amino acid are produced, food, drink, supplement and the like in an unconventional form permitting easy ingestion of amino acid at a high dose can be produced once a solution containing a high concentration of amino acid can be prepared. A higher amino acid concentration of the solution is more preferable, since it is expected to improve easy handling during production, reduce transportation cost and the like.

However, histidine is associated with a problem in that its low water solubility prevents preparation of an aqueous solution having a high concentration.

The aforementioned patent document 1 describes a production method of a food containing 0.1 - 20 wt% of amino acid. However, 17 kinds of amino acids are blended in the sole Example of the document, and the concentration thereof as a concentration of 17 kinds of amino acids is only 1.5 wt%, and the content of histidine in 100 parts by weight of the total amount of the blended 17 kinds of amino acids is only as low as 2 parts by weight.

Accordingly, the present invention aims to provide an aqueous liquid composition containing histidine at a high concentration, which is preferable for ingestion at a high dose, a production method of the liquid composition, use of the liquid composition and the like.

### Means of Solving the Problems

The present inventors have conducted intensive studies in an attempt to achieve the aforementioned object and succeeded for the first time in preparing a liquid composition containing histidine at a concentration exceeding 20 wt%.

While histidine, which is a basic amino acid, shows improved water-solubility under acidity, it was unexpectedly found that the water-solubility of histidine is more remarkably increased in an acidic aqueous solution containing a divalent or higher valent organic acid as an acidic substance than in an acidic aqueous solution containing other acidic substance (e.g., monovalent organic acids such as acetic acid and the like, inorganic acids such as phosphoric acid and the like, and the like) applicable to food and drink, even at the same level of pH.

The present inventors have conducted further studies based on such finding and completed the present invention.

Accordingly, the present invention provides the following.
[1] An aqueous liquid composition comprising histidine at a concentration more than 20 wt%.
[2] The composition of [1], wherein the concentration of histidine is more than 20 wt% and not more than 60 wt%.
[3] An aqueous liquid composition comprising histidine at a concentration more than 20 wt% and a divalent or higher valent organic acid.
[4] The composition of [3], wherein the concentration of histidine is more than 20 wt% and not more than 60 wt%.
[5] The composition of [3] or [4], wherein the divalent or higher valent organic acid is one or more kinds selected from citric acid, malic acid and tartaric acid.
[6] The composition of any of [3] - [5], wherein the content of the divalent or higher valent organic acid per 1 part by weight of histidine in the aqueous liquid composition is not less than (0.0109A+0.0511) parts by weight, when the concentration of histidine in the aqueous liquid composition is A wt%.
[7] A food or drink comprising the composition of any of [1] - [6], wherein histidine is contained in the composition at a concentration other than not more than 20 wt%.
[8] A pharmaceutical product comprising the composition of any of [1] - [6], wherein histidine is contained in the composition at a concentration other than not more than 20 wt%.
[9] A production method of an aqueous liquid composition comprising histidine, comprising a step of dissolving histidine at a concentration of more than 20 wt% in an acidic aqueous solution containing a divalent or higher valent organic acid.
[10] The production method of [9], comprising dissolving histidine at a concentration of more than 20 wt% up to 60 wt%.
[11] The production method of [9] or [10], wherein the divalent or higher valent organic acid is one or more kinds of citric acid, malic acid and tartaric acid.
[12] The production method of any of [9] - [11], wherein the acidic aqueous solution contains not less than (0.0109A+0.0511) parts by weight, when the concentration of histidine in the aqueous liquid composition is A wt%, of the divalent or higher valent organic acid per 1 part by weight of histidine in the aqueous liquid composition.
[13] The production method of any of [9] - [12], wherein the acidic aqueous solution has a pH of not more than 1.0. Effect of the Invention

The aqueous liquid composition provided by the present invention contains histidine at a concentration higher than that of conventionally-known ones, and therefore, can provide food, drink, supplement and the like permitting ingestion of a high dose of histidine in a small amount. The components of the aqueous liquid composition are suitable for oral ingestion, and can provide food, drink, supplement, pharmaceutical product and the like, having superior safety.

When used as a starting material for processing a food or drink, the aqueous liquid composition of the present invention facilitates processing, since it is easily dispersed in the starting material as compared to the addition of histidine as a powder to the starting material.

In addition, when the aqueous liquid composition of the present invention is added to the starting material in the production step of food, drink and pharmaceutical product, histidine does not precipitate even when water content decreases due to heating and drying, and uniformity is maintained. As a result, the histidine concentration of the product is consistent, a rough feeling in the mouth and the like due to precipitation of histidine is not developed, and the constant quality of the product can be maintained.

Furthermore, since the aqueous liquid composition of the present invention is free of histidine precipitation even when stood still under cold storage or freezing, when it is used as a starting material of food, drink, and pharmaceutical product, histidine does not precipitate in the product even if the production and storage steps thereof include refrigeration and freezing, and uniformity is maintained. As a result, the histidine concentration of the product is consistent, a rough feeling in the mouth and the like due to precipitation of histidine is not developed, and superior quality can be maintained.

### Description of Embodiments

The present invention provides an aqueous liquid composition containing histidine at a concentration more than 20 wt%, particularly an aqueous liquid composition further containing a divalent or higher valent organic acid (hereinafter also to be referred to as the aqueous liquid composition of the present invention), and a production method of food or drink containing the same, and the liquid composition (hereinafter also to be referred to as the production method of the present invention).

In the present invention, an aqueous liquid in the aqueous liquid composition means a liquid including water, and is a concept encompassing a liquid consisting only of water, as well as a liquid containing water as a main component and other solvent. Examples thereof include water, alcohol-containing water and the like, and 1 - 100 wt% of water is generally contained.

Histidine to be contained in the aqueous liquid composition of the present invention is not limited as regards its derivation and production method, and any of one obtained by extracting and purifying from naturally-occurring animals, plants and the like, and one obtained by chemical synthesis method, fermentation method, enzyme method or gene recombinant method may be used. As histidine, any of L-form, D-form and DL-form can be used, and L-form is preferably used. Furthermore, as histidine, a free form or a salt (hydrochloride etc.) can be used, and a free form is preferably used, which is added to and dissolved in an acidic aqueous solution.

The concentration of histidine contained in the aqueous liquid composition of the present invention is a concentration more than 20 wt%, for example, not less than 20.5 wt%, preferably not less than 28 wt%, more preferably not less than 30 wt%, further preferably not less than 36 wt%, moreover preferably not less than 40 wt%, and furthermore preferably not less than 45 wt%. When the concentration of histidine is more than 20 wt%, the amount of the aqueous liquid composition necessary for ingesting a desired amount of histidine can be reduced, and histidine can be ingested efficiently.

While the upper limit of the concentration of histidine is not particularly limited as long as histidine is dissolved, it is generally not more than 60 wt%, preferably not more than 57 wt%. When the histidine concentration is not more than 60 wt%, precipitation and sedimentation of histidine is not observed, and a good liquid composition can be obtained.

Furthermore, in the aqueous liquid composition of the present invention, the ratio of the histidine content to the water content (histidine to water content %(W/W)) is not particularly limited as long as histidine is dissolved, and it can be appropriately set within the range of generally about 60 - 900%.

The aqueous liquid composition of the present invention can be prepared by, for example, dissolving histidine in an acidic aqueous solution containing a divalent or higher valent organic acid.

Therefore, the aqueous liquid composition of the present invention generally contains a divalent or higher valent organic acid besides histidine.

The divalent or higher valent organic acid is not limited as to the derivation and production method. Examples of the divalent or higher valent organic acid include, but are not limited thereto, divalent organic acids (e.g., malic acid, tartaric acid, succinic acid, fumaric acid and the like), trivalent organic acids (e.g., citric acid and the like) and the like. One or more kinds of divalent or higher valent organic acids can be selected and used from these. The organic acid is preferably not more than trivalent. Preferred as the divalent or higher valent organic acid is one or more kinds of citric acid, malic acid and tartaric acid. When such divalent or higher valent organic acid is further contained, an aqueous liquid composition containing histidine at a high concentration, which is preferable for oral ingestion, can be produced.

The content of the divalent or higher valent organic acid in the aqueous liquid composition of the present invention is not particularly limited as long as histidine within the range of the aforementioned concentration can stay dissolved in the aqueous liquid composition. The dissolved here means a state free of histidine precipitation or sedimentation when stood still at 20°C for 24 hr. The content of the divalent or higher valent organic acid is not less than (0.0109A+0.0511) parts by weight per 1 part by weight of histidine in the aqueous liquid composition, when the concentration of histidine in the aqueous liquid composition is A wt%. From the data shown in the Examples of the present application, it was found that a relationship represented by the above-mentioned function exists between the concentration of histidine in the aqueous liquid composition and the lower limit of the content ratio (to histidine) of a divalent or higher valent organic acid enabling histidine to stay dissolved in the aqueous liquid composition. That is, when the content of the divalent or higher valent organic acid per 1 part by weight of histidine in the aqueous liquid composition is not less than (0.0109A+0.0511) parts by weight, histidine at a concentration more than 20 wt% can stay dissolved in the aqueous liquid composition.

For example, when the concentration of histidine in the aqueous liquid composition is 20.5 wt%, histidine can stay dissolved in the aqueous liquid composition by setting the content of divalent or higher valent organic acid in the aqueous liquid composition to not less than about 0.27 parts by weight per 1 part by weight of histidine.

For example, when the concentration of histidine in the aqueous liquid composition is 30 wt%, histidine can stay dissolved in the aqueous liquid composition by setting the content of divalent or higher valent organic acid in the aqueous liquid composition to not less than about 0.38 parts by weight per 1 part by weight of histidine.

For example, when the concentration of histidine in the aqueous liquid composition is 40 wt%, histidine can stay dissolved in the aqueous liquid composition by setting the content of divalent or higher valent organic acid in the aqueous liquid composition to not less than about 0.49 parts by weight per 1 part by weight of histidine.

For example, when the concentration of histidine in the aqueous liquid composition is 45 wt%, histidine can stay dissolved in the aqueous liquid composition by setting the content of divalent or higher valent organic acid in the aqueous liquid composition to not less than about 0.54 parts by weight per 1 part by weight of histidine.

From the aspect of enhancing the content of histidine in the aqueous liquid composition of the present invention, the content of the divalent or higher valent organic acid is generally not more than 2.0 parts by weight, preferably not more than 1.2 parts by weight, more preferably not more than 1.0 part by weight, and furthermore preferably not more than 0.8 part by weight, per 1 part by weight of histidine. The contents of histidine and divalent or higher valent organic acid are appropriately determined within the range where the total concentration thereof in the aqueous liquid composition of the present invention is less than 100 wt%.

The aqueous liquid composition of the present invention may contain, as necessary, a component other than histidine and organic acid. Examples of other component include, but are not particularly limited to, starting materials generally used in the production of food, drink, pharmaceutical product and the like. Examples thereof include thickener, sweetening agent, corrigent, preservative, flavor, excipient, stabilizer and the like.

Examples of the thickener include polymers such as dextrin, sodium alginate, propylene glycol alginate, powdered tragacanth, xanthan gum, sodium carboxymethylcellulose, hydroxypropylcellulose, polyvinyl alcohol, polyvinylpyrrolidone and the like.

Examples of the sweetening agent include glucose, fructose, invert sugar, sorbitol, xylitol, glycerol, simple syrup and the like. In addition, to provide histidine at a high concentration by reducing the contents of other components, a high intensity sweetener can also be used. Examples of the high intensity sweetener include aspartame (α-L-aspartyl phenylalanine methyl ester), acesulfame K (6-methyl-1,2,3-oxathiazine-4(3H)-one-2,2-dioxide potassium), sucralose (4,1,6-trichlorogalacto sucrose), glycyrrhizin, thaumatin, monellin and the like.

Examples of the corrigent include aspartame, saccharin, saccharin sodium, glycyrrhizic acid, monoammonium glycyrrhizinate, diammonium glycyrrhizinate, dipotassium glycyrrhizinate, trisodium glycyrrhizinate, acesulfame K, mannitol, erythritol, sorbitol, xylitol, trehalose, cacao powder and the like.

Examples of the preservative include middle chain fatty acid monoglyceride, glycine, ethanol and the like.

Examples of the flavor include lemon flavor, orange flavor, grapefruit flavor, chocolate flavor, apple flavor, dl-menthol, 1-menthol and the like.

Examples of the excipient include alcohol, glycerol, invert sugar, glucose, vegetable oil, wax, fat, semisolid and liquid polyol and the like.

Examples of the stabilizer include acetic acid, sodium bisulfite, sodium thiosulfate, sodium edetate, ascorbic acid, dibutylhydroxytoluene and the like.

The production method of the aqueous liquid composition of the present invention (hereinafter to be also referred to as the production method of the present invention) is now explained. The following production method shows one example of the present invention, is not particularly limited, and can be appropriately changed and modified by those of ordinary skill in the art when desired.

The production method of the present invention includes, for example, a step of dissolving histidine in an acidic aqueous solution containing a divalent or higher valent organic acid.

The acidic aqueous solution containing a divalent or higher valent organic acid is an acidic aqueous solution containing the aforementioned divalent or higher valent organic acid (preferably one or more kinds of citric acid, malic acid and tartaric acid). The solvent used to dissolve the divalent or higher valent organic acid is not particularly limited as long as it is generally used in the production of food and drink, and the aforementioned aqueous liquids are generally used.

A divalent or higher valent organic acid is added to the aforementioned solvent to achieve the above-mentioned content in the aqueous liquid composition of the present invention, and the mixture is appropriately stirred for dissolution to give an acidic aqueous solution. The solvent is preferably heated to promote dissolution of the divalent or higher valent organic acid and, for example, the temperature thereof is preferably 80 - 100°C in the case of water.

While adjustment of the pH of the acidic aqueous solution is not necessary since the pH decreases when a divalent or higher valent organic acid is dissolved in a solvent as described above, the pH is generally not more than 5, preferably not more than 3, more preferably not more than 1.

Histidine is added to the acidic aqueous solution prepared as mentioned above to the aforementioned content in the aqueous liquid composition of the present invention, and the mixture is appropriately dissolved by stirring. The acidic aqueous solution is preferably heated to promote dissolution of histidine. For example, when an acidic aqueous solution is used, the temperature thereof is preferably 80 - 100°C.

When histidine is dissolved in a heated acidic aqueous solution, the solubility of histidine may be confirmed by allowing same to cool and be stood still at 20°C for 24 hr. When histidine and a divalent or higher valent organic acid are added at the aforementioned contents, the solubility of histidine is fine and precipitation and sedimentation of histidine are not found.

The components other than histidine and organic acid in the aqueous liquid composition of the present invention (e.g., the aforementioned thickener, sweetening agent, corrigent, preservative, flavor, excipient, stabilizer and the like) can be added at any stage and, for example, may be added to an acidic aqueous solution or after addition of histidine.

The aqueous liquid composition of the present invention by itself can be provided as food, drink, pharmaceutical product and the like. In addition, it is useful as a starting material for producing food, drink, pharmaceutical product and the like containing histidine at any concentration. When the aqueous liquid composition of the present invention is utilized as a starting material, it may be utilized after dilution to any histidine concentration of not more than 20 wt%. Using the aqueous liquid composition of the present invention or a diluted solution thereof, food, drink, pharmaceutical product and the like can be produced more easily than by direct handling of solid histidine.

Since histidine is involved in growth, plays a role of nerve function support, and further has an antioxidant action and a stress suppressive action, the aqueous liquid composition of the present invention can be applied to food, drink and pharmaceutical product for the improvement of the symptoms of chronic arthritis, reduction of stress, improvement of sexual desire, enhancement of concentration and memory, prophylaxis of obesity, antifatigue effect, and the like.

When used as a starting material to be processed into food and drink, the aqueous liquid composition of the present invention is useful for production and preservation from the following aspects.
(1) In the production of food, drink, and pharmaceutical product, when histidine is added to a starting material, histidine in the form of a liquid is easily dispersed in the starting material as compared to the use of a powder. Particularly, the effect is more prominent when used in a system containing less water and a system using a starting material having high viscosity.
(2) When a histidine liquid is added to the starting material in the production step of food, drink and pharmaceutical product, histidine does not precipitate even when water content decreases due to heating and drying, and uniformity is maintained. As a result, the histidine concentration of the product is consistent, a rough feeling in the mouth and the like due to precipitation of histidine is not developed.
(3) The liquid is free of histidine precipitation even when stood still under cold storage or freezing. Therefore, when histidine in the form of a liquid is used as a starting material of food, drink, and pharmaceutical product, histidine does not precipitate in the product even if the production and storage steps thereof include refrigeration and freezing, and uniformity is maintained. As a result, the histidine concentration of the product is consistent, a rough feeling in the mouth and the like due to precipitation of histidine is not developed.

In addition, the aqueous liquid composition of the present invention can be used for the production of the above-mentioned food, drink, pharmaceutical product and the like, as well as food and drink containing the aqueous liquid composition of the present invention containing histidine at a concentration other than not more than 20 wt%.

The present invention further provides food and drink containing such aqueous liquid composition of the present invention. In addition, it also provides a pharmaceutical product containing the aqueous liquid composition.

In the present specification, the "food and drink" means foods in general including drinks, and also encompasses specific-use food (food for sick person, food for specified health uses) defined in the specific-use food system by the Consumer Affairs Agency, food for specified health uses and food with nutrient function claims defined in the food with health claims system, and further, dietary supplements (nutrition assistant food).

While the food and drink of the present invention is not particularly limited as long as it is in a form permitting oral ingestion, examples thereof include foods such as drink, jelly, gummi, candy, gum, chocolate and the like, nutrition food, capsule and the like. Of these forms, a capsule is preferable since histidine at a high dose can be conveniently ingested. They can be prepared according to a method known in the field, by mixing the liquid composition of the present invention in a jelly form or enclosing the liquid composition in a suitable capsule, so that the aqueous liquid composition will not be mixed with other starting materials and diluted to a concentration of not more than 20 wt%.

The food and drink of the present invention contains the aqueous liquid composition of the present invention containing histidine at a concentration other than not more than 20 wt%.. Preferably, the aqueous liquid composition of the present invention and other starting materials exist in clearly separated states in the food and drink of the present invention. Examples of such food and drink include, but are not limited to, capsule enclosing the aqueous liquid composition of the present invention, jelly, gummi, candy, gum, and chocolate, a food having a high fat and oil content, less water, a high concentration of histidine in the aqueous phase, and the aqueous phase dispersed and emulsified in the fat and oil component (e.g., mayonnaise, dressing, margarine, cream, seasoning oil and the like) and the like.

While the content of the aqueous liquid composition of the present invention in the food and drink of the present invention is not particularly limited, it can be appropriately determined such that a desired amount of histidine can be ingested from the food and drink of the present invention.

The pharmaceutical product of the present invention can be formulated as a pharmaceutical composition according to conventional means, such that the aqueous liquid composition of the present invention is not diluted to a concentration of not more than 20 wt% by mixing with other starting materials. The pharmaceutical product of the present invention can be administered orally or parenterally to a subject directly as a liquid, or a pharmaceutical product in a suitable dosage form. The pharmaceutical product of the present invention is generally administered orally.

The aqueous liquid composition of the present invention is contained such that a desired amount of histidine is contained in the pharmaceutical product of the present invention. The content thereof is generally about 0.1 - 100 wt%, preferably about 1 - 99 wt%, more preferably about 10 - 90 wt%, relative to the whole pharmaceutical composition.

The food, drink, and pharmaceutical product of the present invention are useful since a high dose of histidine can be ingested by taking a small amount.

The present invention is further explained in detail in the following by referring to Examples; however, the present invention is not limited by the following Examples. Examples

### Example 1: Influence of the kind of acidic substance on solubility of histidine

Acidic substances (acetic acid, phosphoric acid, citric acid, malic acid or tartaric acid) in the amounts indicated in Table 1 were added to and dissolved in water at 80 - 100°C to prepare acidic aqueous solutions. The pH of the acidic aqueous solutions was each not more than 1.20 as shown in Table 1. Then, to the prepared acidic aqueous solutions was added histidine (HIS) in the amounts indicated in Table 1, and the mixtures were thoroughly stirred. The mixtures were left standing at room temperature for 24 hr, and the solubility of histidine was examined.

**Table 1**

| | | acetic acid | acetic acid | acetic acid | phosphoric acid | citric acid | citric acid | citric acid | malic acid | tartaric acid |
|---|---|---|---|---|---|---|---|---|---|---|
| molecular weight | | 60.5 | 60.5 | 60.5 | 98 | 210.14 | 210.14 | 210.14 | 134.09 | 150 |
| valence | | 1 | 1 | 1 | 3 | 3 | 3 | 3 | 2 | 2 |
| water | (g) | 50 | 20 | 50 | 50 | 50 | 50 | 50 | 50 | 50 |
| His | (g) | 90 | 60 | 35 | 90 | 90 | 100 | 120 | 90 | 90 |
| | (mol) | 0.58 | 0.39 | 0.23 | 0.58 | 0.58 | 0.64 | 0.77 | 0.58 | 0.58 |
| organic acid | (g) | 225 | 90 | 60 | 60 | 60 | 65 | 80 | 60 | 60 |
| | (mol) | 3.7 | 1.5 | 1.0 | 0.6 | 0.29 | 0.31 | 0.38 | 0.45 | 0.4 |
| acidic substance/HIS | (W/W) | 2.50 | 1.50 | 1.71 | 0.67 | 0.67 | 0.65 | 0.67 | 0.67 | 0.67 |
| | (mol/mol) | 6.46 | 3.88 | 4.43 | 1.06 | 0.49 | 0.48 | 0.49 | 0.77 | 0.69 |
| pH (before His addition) | | 0.3 | 0.3 | 1.20 | less than 0.10 | 0.40 | 0.30 | 0.10 | 0.40 | 0.20 |
| HIS concentration %(W/W) in liquid | | 24.7 | 35.3 | 24.1 | 45.0 | 45.0 | 46.5 | 48.0 | 45.0 | 45.0 |
| HIS content (%) to water (W/W) | | 180 | 300 | 70 | 180 | 180 | 200 | 240 | 180 | 180 |
| solubility | | × | × | × | × | ○ | ○ | ○ | ○ | ○ |

The results are shown in Table 1. When an aqueous liquid composition having pH of not more than 1.0 was prepared by using citric acid, malic acid or tartaric acid as an acidic substance, the histidine added was finely dissolved even in an amount corresponding to the concentration in the composition of not less than 45 wt% (in Table 1, solubility ○), and a liquid composition containing histidine at a high concentration could be prepared. On the other hand, when an acidic aqueous solution having pH of not more than 1.0 was prepared by using acetic acid or phosphoric acid as an acidic substance, the histidine added was not dissolved or, even when dissolved, precipitated after standing at room temperature for 24 hr (in Table 1, solubility x), and a liquid composition containing histidine at a high concentration could not be prepared.

These results reveal that a liquid composition containing histidine at a high concentration cannot be prepared by merely deceasing the pH of the acidic aqueous solution to a given value or lower, and a divalent or higher valent organic acid such as citric acid, malic acid, tartaric acid and the like needs to be used.

### Example 2: Influence of addition ratio of acidic substance to histidine (when preparing liquid composition containing 45 wt% or more of histidine)

Acidic substances (citric acid, malic acid or tartaric acid) in the amounts indicated in Table 2 were added to and dissolved in water at 80 - 100°C to prepare acidic aqueous solutions. Then, to the prepared acidic aqueous solutions was added histidine (HIS) in the amounts indicated in Table 2 to a concentration of not less than 45 wt%, and the mixtures were thoroughly stirred. The mixtures were left standing at room temperature for 24 hr, and the solubility of histidine was examined. In Table 2, "-" in the pH column means unmeasurable. In Table 2, the composition with *2 was prepared after preparation of the composition with *1, by reducing the amount of water by evaporation under reduced pressure.

**Table 2**

| | | citric acid | citric acid | citric acid | citric acid | citric acid | citric acid | citric acid | malic acid | malic acid | malic acid | tartaric acid | tartaric acid |
|---|---|---|---|---|---|---|---|---|---|---|---|---|---|
| molecular weight | | 210.14 | 210.14 | 210.14 | 210.14 | 210.14 | 210.14 | 210.14 | 134.09 | 134.09 | 134.09 | 150 | 150 |
| valence | | 3 | 3 | 3 | 3 | 3 | 3 | 3 | 2 | 2 | 2 | 2 | 2 |
| water | (g) | 50 | 50 | 50 | 50 | 50 | 20 | 10 | 50 | 50 | 50 | 50 | 50 |
| His | (g) | 90 | 90 | 100 | 120 | 180 | 90 | 90 | 90 | 90 | 100 | 90 | 100 |
| | (mol) | 0.58 | 0.58 | 0.64 | 0.77 | 1.16 | 0.58 | 0.58 | 0.58 | 0.58 | 0.64 | 0.58 | 0.64 |
| organic acid | (g) | 60 | 50 | 65 | 80 | 120 | 60 | 60 | 50 | 60 | 70 | 60 | 60 |
| | (mol) | 0.29 | 0.24 | 0.31 | 0.38 | 0.57 | 0.29 | 0.29 | 0.37 | 0.45 | 0.52 | 0.4 | 0.4 |
| acidic substance/HIS | (W/W) | 0.67 | 0.56 | 0.65 | 0.67 | 0.67 | 0.67 | 0.67 | 0.56 | 0.67 | 0.70 | 0.67 | 0.60 |
| | (mol/mol) | 0.49 | 0.41 | 0.48 | 0.49 | 0.49 | 0.49 | 0.49 | 0.64 | 0.77 | 0.81 | 0.69 | 0.62 |
| pH (before His addition) | | 0.40 | - | 0.30 | 0.10 | - | - | - | - | 0.40 | - | 0.20 | 0.20 |
| HIS concentration %(W/W) in liquid | | 45.0 | 47.4 | 46.5 | 48.0 | 51.4 | 52.9 | 56.3 | 47.4 | 45.0 | 45.5 | 45.0 | 47.6 |
| HIS content relative to water % (W/W) | | 180 | 180 | 200 | 240 | 360 | 450 | 900 | 180 | 180 | 200 | 180 | 200 |
| solubility | | ○ | × | ○ | ○ | ○ | ○ | ○ | × | ○ | ○ | ○ | ○ |
| | | *1 | | | | | *2 | *2 | | | | | |

The results are shown in Table 2. When an aqueous liquid composition was prepared by using not less than 0.65 part by weight of citric acid, not less than 0.67 part by weight of malic acid, or not less than 0.60 part by weight of tartaric acid, per 1 part by weight of histidine, a liquid composition having a histidine concentration of not less than 45 wt% could be prepared. On the other hand, when an acidic aqueous solution was prepared by using 0.56 part by weight of citric acid or malic acid per 1 part by weight of histidine, a liquid composition having a histidine concentration of not less than 47.4 wt% could not be prepared.

In Table 2, histidine did not precipitate, the composition with *2 maintained solubility and uniformity in the liquid even when water was evaporated from the composition of *1 (histidine high concentration liquid) and the concentration of histidine in the liquid increased.

Even when the composition with *1 (histidine high concentration liquid) was placed under a freezing temperature (-80°C, 24 hr), histidine did not precipitate, and the solubility and uniformity of the liquid were maintained. When the composition was allowed to warm to room temperature, the properties did not change from those before freezing.

### Example 3: Influence of amounts of addition of water, acidic substance and histidine

Citric acid in the amount indicated in Tables 3 - 5 were added to and dissolved in water at 80 - 100°C to prepare acidic aqueous solutions. Then, to the prepared acidic aqueous solutions was added histidine (HIS) in the amounts indicated in Tables 3 - 5, and the mixtures were thoroughly stirred. The mixtures were left standing at room temperature for 24 hr, and the solubility of histidine was examined. In Table 5, the composition with *2 was prepared after preparation of the composition with *1, by reducing the amount of water by evaporation under reduced pressure.

**Table 3**

| | | citric acid | citric acid | citric acid | citric acid |
|---|---|---|---|---|---|
| molecular weight | | 210.14 | 210.14 | 210.14 | 210.14 |
| valence | | 3 | 3 | 3 | 3 |
| water | (g) | 50 | 50 | 50 | 50 |
| His | (g) | 90 | 70 | 50 | 30 |
| | (mol) | 0.58 | 0.45 | 0.32 | 0.19 |
| organic acid | (g) | 60 | 60 | 60 | 60 |
| | (mol) | 0.29 | 0.29 | 0.29 | 0.29 |
| acidic substance/HIS | (W/W) | 0.67 | 0.86 | 1.20 | 2.00 |
| | (mol/mol) | 0.49 | 0.63 | 0.89 | 1.48 |
| HIS concentration in liquid | % (W/W) | 45.0 | 38.9 | 31.3 | 21.4 |
| HIS content relative to water % (W/W) | | 180 | 140 | 100 | 60 |
| solubility | | ○ | ○ | ○ | ○ |

**[Table 4]**

| | | citric acid | citric acid | citric acid | citric acid |
|---|---|---|---|---|---|
| molecular weight | | 210.14 | 210.14 | 210.14 | 210.14 |
| valence | | 3 | 3 | 3 | 3 |
| water | (g) | 50 | 50 | 50 | 50 |
| His | (g) | 90 | 70 | 50 | 30 |
| | (mol) | 0.58 | 0.45 | 0.32 | 0.19 |
| organic acid | (g) | 60 | 46.7 | 33.3 | 20.0 |
| | (mol) | 0.29 | 0.22 | 0.16 | 0.10 |
| acidic substance/HIS | (W/W) | 0.67 | 0.67 | 0.67 | 0.67 |
| | (mol/mol) | 0.49 | 0.49 | 0.49 | 0.49 |
| HIS concentration in liquid | % (W/W) | 45.0 | 42.0 | 37.5 | 30.0 |
| HIS content relative to water % (W/W) | | 180 | 140 | 100 | 60 |
| solubility | | ○ | ○ | ○ | ○ |

**[Table 5]**

| | | citric acid | citric acid | citric acid | citric acid |
|---|---|---|---|---|---|
| molecular weight | | 210.14 | 210.14 | 210.14 | 210.14 |
| valence | | 3 | 3 | 3 | 3 |
| water | (g) | 50 | 38.9 | 27.8 | 16.7 |
| His | (g) | 90 | 70 | 50 | 30 |
| | (mol) | 0.58 | 0.45 | 0.32 | 0.19 |
| organic acid | (g) | 60 | 60 | 60 | 60 |
| | (mol) | 0.29 | 0.29 | 0.29 | 0.29 |
| acidic substance/HIS | (W/W) | 0.67 | 0.86 | 1.20 | 2.00 |
| | (mol/mol) | 0.49 | 0.63 | 0.89 | 1.48 |
| HIS concentration % (W/W) in liquid | | 45.0 | 41.4 | 36.3 | 28.1 |
| HIS content relative to water % (W/W) | | 180 | 180 | 180 | 180 |
| solubility | | ○ | ○ | ○ | ○ |
| | | | | *1 | *2 |

As shown in Table 3, even when the amount of addition of histidine added to the acidic aqueous solution (water 50 g, citric acid 60 g) was reduced, the solubility of histidine was fine.

As shown in Table 4, when the amounts of addition of histidine and acidic substance added were reduced to achieve a constant ratio of histidine and acidic substance (acidic substance/HIS=0.67(w/w)), the solubility of histidine was fine.

Moreover, as shown in Table 5, when the amounts of addition of histidine and water were reduced to achieve a constant ratio of histidine and water (histidine content to water=180% (w/w)), the solubility of histidine was fine.

### Example 4: Influence of addition ratio of acidic substance to histidine (when preparing liquid composition containing 20 wt% or 30 wt% of histidine)

Acidic substances (citric acid or malic acid) in the amounts indicated in Table 6 or 7 were added to and dissolved in water at 80 - 100°C to prepare acidic aqueous solutions. Then, to the prepared acidic aqueous solutions was added histidine in the amounts indicated in Table 6 or 7 to a concentration of 20 wt% or 30 wt%, and the mixtures were thoroughly stirred. The mixtures were left standing at room temperature for 24 hr, and the solubility of histidine was examined.

**[Table 6]**

| | | citric acid | citric acid | citric acid | citric acid | citric acid | malic acid | citric acid |
|---|---|---|---|---|---|---|---|---|
| molecular weight | | 210.14 | 210.14 | 210.14 | 210.14 | 210.14 | 150 | 210.14 |
| valence | | 3 | 3 | 3 | 3 | 3 | 2 | 3 |
| water | (g) | 33.3 | 34.3 | 35.3 | 36.3 | 37.3 | 37.3 | 38.3 |
| His | (g) | 10 | 10 | 10 | 10 | 10 | 10 | 10 |
| | (mol) | 0.06 | 0.06 | 0.06 | 0.06 | 0.06 | 0.06 | 0.06 |
| organic acid | (g) | 6.7 | 5.7 | 4.7 | 3.7 | 2.7 | 2.7 | 1.7 |
| | (mol) | 0.032 | 0.027 | 0.022 | 0.018 | 0.013 | 0.013 | 0.008 |
| acidic substance/HIS | (W/W) | 0.67 | 0.57 | 0.47 | 0.37 | 0.27 | 0.27 | 0.17 |
| | (mol/mol) | 0.49 | 0.42 | 0.35 | 0.27 | 0.20 | 0.20 | 0.13 |
| HIS concentration % liquid | (W/W) in | 20.0 | 20.0 | 20.0 | 20.0 | 20.0 | 20.0 | 20.0 |
| HIS content relative to water % (W/W) | | 30.0 | 29.2 | 28.3 | 27.5 | 26.8 | 26.8 | 26.1 |
| solubility | | ○ | ○ | ○ | ○ | ○ | ○ | × |

**[Table 7]**

| | | citric acid | citric acid | citric acid | citric acid | citric acid | malic acid | citric acid |
|---|---|---|---|---|---|---|---|---|
| molecular weight | | 210.14 | 210.14 | 210.14 | 210.14 | 210.14 | 150 | 210.14 |
| valence | | 3 | 3 | 3 | 3 | 3 | 2 | 3 |
| water | (g) | 25.0 | 26.0 | 27.0 | 28.0 | 29.0 | 29.0 | 30.0 |
| His | (g) | 15 | 15 | 15 | 15 | 15 | 15 | 15 |
| | (mol) | 0.10 | 0.10 | 0.10 | 0.10 | 0.10 | 0.10 | 0.10 |
| organic acid | (g) | 10.0 | 9.0 | 8.0 | 7.0 | 6.0 | 6.0 | 5.0 |
| | (mol) | 0.048 | 0.043 | 0.038 | 0.033 | 0.029 | 0.040 | 0.024 |
| acidic substance/HIS | (W/W) | 0.67 | 0.60 | 0.53 | 0.47 | 0.40 | 0.40 | 0.33 |
| | (mol/mol) | 0.49 | 0.44 | 0.39 | 0.34 | 0.30 | 0.41 | 0.25 |
| HIS concentration % liquid | (W/W) in | 30.0 | 30.0 | 30.0 | 30.0 | 30.0 | 30.0 | 30.0 |
| HIS content relative to water % (W/W) | | 60 | 57.7 | 55.6 | 53.6 | 51.7 | 51.7 | 50 |
| solubility | | ○ | ○ | ○ | ○ | ○ | ○ | × |

As shown in Table 6, when histidine was added at a concentration of 20 wt%, histidine was not dissolved even when 0.17 part by weight of an acidic substance was added per 1 part by weight of histidine. When not less than 0.27 part by weight of the acidic substance was added per 1 part by weight of histidine, the solubility of histidine was fine.

As shown in Table 7, moreover, when histidine was added at a concentration of 30 wt%, histidine was not dissolved even when 0.33 part by weight of the acidic substance was added per 1 part by weight of histidine. On the other hand, when not less than 0.40 part by weight of the acidic substance was added per 1 part by weight of histidine, the solubility of histidine was fine.

In the data obtained using divalent or higher valent organic acids in the above Examples 1 - 4, when "HIS concentration % (W/W) in liquid" as x and "acidic substance/HIS (W/W)" as y are plotted on a graph, the composition that afforded good histidine solubility (in Table, ○) and the composition that failed to afford good histidine solubility (in Table, x) were clearly distinguished by a line shown by the function (y=0.0109x+0.0511) on the xy plane. Therefore, it was clarified that a liquid composition containing histidine at a high concentration can be prepared by setting "HIS concentration % (W/W) in liquid" and "acidic substance/HIS (W/W)" to fall within the area on the xy plane, which includes those that could afford good histidine solubility in the Example.

### Industrial Applicability

Since the present invention provides a liquid composition containing histidine at a concentration higher than that of conventionally-known ones, it is useful in the fields of food and drink, food with health claims, specific-use food, pharmaceutical product and the like.

This application is based on patent application No. 2013-045654 filed in Japan, the contents of which are encompassed in full herein.

## Claims

1. An aqueous liquid composition comprising histidine at a concentration more than 20 wt%.

2. The composition according to claim 1, wherein the concentration of histidine is more than 20 wt% and not more than 60 wt%.

3. An aqueous liquid composition comprising histidine at a concentration more than 20 wt% and a divalent or higher valent organic acid.

4. The composition according to claim 3, wherein the concentration of histidine is more than 20 wt% and not more than 60 wt%.

5. The composition according to claim 3 or 4, wherein the divalent or higher valent organic acid is one or more kinds selected from citric acid, malic acid and tartaric acid.

6. The composition according to any one of claims 3 to 5, wherein the content of the divalent or higher valent organic acid per 1 part by weight of histidine in the aqueous liquid composition is not less than (0.0109A+0.0511) parts by weight, when the concentration of histidine in the aqueous liquid composition is A wt%.

7. A food or drink comprising the composition according to any one of claims 1 to 6, wherein histidine is contained in the composition at a concentration other than not more than 20 wt%.

8. A pharmaceutical product comprising the composition according to any one of claims 1 to 6, wherein histidine is contained in the composition at a concentration other than not more than 20 wt%.

9. A production method of an aqueous liquid composition comprising histidine, comprising a step of dissolving histidine at a concentration of more than 20 wt% in an acidic aqueous solution containing a divalent or higher valent organic acid.

10. The production method according to claim 9, comprising dissolving histidine at a concentration of more than 20 wt% up to 60 wt%.

11. The production method according to claim 9 or 10, wherein the divalent or higher valent organic acid is one or more kinds of citric acid, malic acid and tartaric acid.

12. The production method according to any one of claims 9 to 11, wherein the acidic aqueous solution contains not less than (0.0109A+0.0511) parts by weight, when the concentration of histidine in the aqueous liquid composition is A wt%, of the divalent or higher valent organic acid per 1 part by weight of histidine in the aqueous liquid composition.

13. The production method according to any one of claims 9 to 12, wherein the acidic aqueous solution has a pH of not more than 1.0.
